# NEUE EUROPÄISCHE PATENTSCHRIFT

(11) **EP 2 742 465 B2**
(45) Veröffentlichungstag und Bekanntmachung des Hinweises auf die Entscheidung über den Einspruch: **25.12.2019**
(45) Hinweis auf die Patenterteilung: 12.04.2017
(21) Anmeldenummer: 12750722.6
(22) Anmeldetag: 06.08.2012
(51) Int. Cl.: A61B 90/90, A61B 90/98

(54) **RFID-TAG**
RFID TAG
ÉTIQUETTE RFID

(30) Priorität: 08.08.2011 DE 102011052501
(43) Veröffentlichungstag der Anmeldung: 18.06.2014
(60) Teilanmeldung: 17156719.1
(73) Patentinhaber: Aesculap AG, 78532 Tuttlingen (DE)
(72) Erfinder: WEIßHAUPT, Dieter, 78194 Immendingen (DE); MORALES, Pedro, 78532 Tuttlingen-Nendingen (DE)
(74) Vertreter: Hoeger, Stellrecht & Partner Patentanwälte mbB
(86) Internationale Anmeldenummer: PCT/EP2012/065324
(87) Internationale Veröffentlichungsnummer: WO 2013/020944

(56) Entgegenhaltungen:
- EP-A1- 0 619 101
- EP-A1- 1 704 893
- EP-A2- 2 062 525
- WO-A1-00/21031
- WO-A1-00/21031
- WO-A1-2008/112709
- WO-A2-2006/020377
- WO-A2-2007/125292
- WO-A2-2010/145651
- US-A- 6 121 544
- US-A1- 2002 060 629
- US-A1- 2006 084 934
- US-A1- 2006 283 945
- US-A1- 2009 295 036
- US-A1- 2010 272 532
- US-A1- 2010 288 843
- US-A1- 2011 057 854
- US-B1- 6 366 206
- US-B1- 7 722 531
- MEL M. SCHWARTZ: "Brazing", 2003, ISBN: 0-87170-784-5, article "Chapter 1", pages: 1 - 5
- Bloss Matthew C.: "Ultrasonic metal welding: the weldability of stainless steel, titanium, and nickel-based", A THESIS PRESENTED IN PARTIAL FULFILLMENT OF THE REQUIREMENTS FOR THE DEGREE MASTER OF SCIENCE IN THE GRADUATE SCHOOL OF THE OHIO STATE UNIVERSITY, 2008, pages 1 - 248
- DENNY ET AL., LASER BEAM WELDING OF TITANIUM, August 1989 (1989-08-01)
- Wikipedia-Artikel über Spritzgießen vom 12 Sept. 20??, aus dem Internet archiveweb. archive.org

## Beschreibung

Die Erfindung betrifft ein RFID-Markierungselement zur Bestückung chirurgischer Instrumente, im Folgenden kurz Tag genannt.

Herkömmliche RFID-Tags zur Bestückung chirurgischer Instrumente umfassen neben einer im Wesentlichen ringförmigen Metallfassung einen scheibenförmigen Kunststoff- oder Keramikkörper, der von der Metallfassung umgeben wird. Die Metallfassung selbst kann durch Schweißen, insbesondere Laserschweißen, an einem zu bestückenden Instrument befestigt werden.

Bei anderen Lösungen wird ein RFID-Element in einer Bohrung im Instrument vergossen.

Die Vorteile der mit RFID-Tags bestückten chirurgischen Instrumente liegen in deren einfacher Identifizierbarkeit und Nachverfolgbarkeit, wobei die RFID-Tags ohne besondere Kenntnisse des Personals überprüft und ausgelesen werden können.

Mit den RFID-Tags bestückte Instrumente mindern das Risiko, dass Instrumente nach einer Operation im Körper des Patienten verbleiben. Auch ist das Instrumenteninventar leichter zu verwalten und die Verwendung der chirurgischen Instrumente während ihrer Lebensdauer besser nachzuvollziehen.

Bei beiden bekannten Lösungen besteht das Problem, dass die Sendeleistung des RFID-Elements des RFID-Tags vergleichsweise gering ist.

Aus der WO 2008/112709 A1 ist ein Transponder bekannt, dessen Gehäuse mittels eines Metallbandes an einem zu markierenden chirurgischen Instrument befestigt werden kann. Aus der EP 1 704 893 A1 ist ein RFID-Detektions- und Identifizierungssystem für implantierbare medizinische Vorrichtungen bekannt.

Bei den unter dem Namen SIMSAFE bekannten RFID-Tags wird eine ringförmige Metallfassung verwendet, in der ein Schlitz angebracht ist, um einen Kurzschluss zu verhindern. Trotzdem sind die Sendeleistungen der RFID-Tags solchermaßen bestückter Instrumente nach wie vor für eine einfache Handhabung im Klinikalltag zu gering.

Aufgabe der Erfindung ist es, einen RFID-Tag vorzuschlagen, der zur Bestückung chirurgischer Instrumente geeignet ist und mit dem neben einer stabilen Verankerung am Instrument, einer Verkapselung der RFID-Elemente gegenüber der Umgebung und einer guten Reinigbarkeit auch eine verbesserte Detektierbarkeit, d.h. Sendeleistung im Umfeld des Instruments, erzielt werden kann.

Erfindungsgemäß wird diese Aufgabe durch ein RFID-Tag wie in Anspruch 1 definiert gelöst.

Anders als bei den bisher bekannten RFID-Tags ist bei den erfindungsgemäßen RFID-Tags ein Gehäuse vorgesehen, welches nur mit einem ersten Ende an der Metallfassung gehalten ist. Die Aufnahmekammer für das RFID-Element ist in dem Gehäuse von dessen erstem Ende räumlich beabstandet ausgebildet. Dies ermöglicht eine Anordnung des RFID-Elements mit seiner Antenne derart, dass die Antenne räumlich im Wesentlichen außerhalb der Metallfassung angeordnet ist. Die Antenne ist in dem Gehäuse vorzugsweise so angeordnet, dass sich ein räumlicher Abstand zu der Metallfassung ergibt.

Die erfindungsgemäßen RFID-Tags ermöglichen neben der stabilen Verankerung des Tags am Instrument eine sichere Kapselung des RFID-Elements, eine gute Reinigbarkeit und darüber hinaus eine Positionierung des RFID-Elements im Gehäuse, die eine im Wesentlichen ungehinderte Abstrahlung des RFID-Elements zulässt.

Darüber hinaus ist eine große Flexibilität für die Auswahl der Stelle am Instrument, an der das RFID-Tag positioniert werden soll, gegeben.

Das Gehäuse mit seiner Aufnahmekammer kann als einfache Verkapselung ausgeführt sein, wobei das RFID-Element in das Gehäusematerial eingegossen ist. Ein gesonderter Fertigungsschritt zur Ausbildung der Aufnahmekammer erübrigt sich bei dieser Ausführungsform.

Bevorzugte Materialien zur Herstellung des Gehäuses bzw. der Verkapselung sind Kunststoff- oder keramische Materialien.

Die RFID-Elemente werden bevorzugt in einer zuvor gefertigten Aufnahmekammer des Gehäuses eingesetzt und dort beispielsweise kraft- und/oder formschlüssig oder auch stoffschlüssig mit dem Gehäuse verbunden gehalten.

Gemäß einer bevorzugten Variante der erfindungsgemäßen RFID-Tags ist als Gehäuse ein einseitig offenes Gehäuse vorgesehen, wobei die Öffnung des Gehäuses einen Zugang zu der Aufnahmekammer bildet. Die Öffnung wird dabei so gestaltet, dass das RFID-Tag in das Gehäuse bzw. dessen Aufnahmekammer einfach eingesetzt werden kann. Die Aufnahmekammer ist in ihrem Querschnitt vorzugsweise so an die Geometrie des RFID-Elements angepasst, dass dieses mit einer Führung durch die Wände der Kammer in seine endgültige Montageposition eingerückt werden kann.

Die Aufnahmekammer als einseitig offene Kammer ist mit einem Verschlusselement verschließbar, wobei das Verschlusselement vorzugsweise mit dem Gehäuse verklebt oder verschweißt, insbesondere mittels Ultraschall verschweißt wird.

Bei einer alternativen bevorzugten Ausführungsform der Erfindung umfasst das erste Ende des Gehäuses die den Zugang zur Aufnahmekammer bildende Öffnung. Die Metallfassung kann dann so gestaltet werden, dass das erste Ende, das die den Zugang zur Aufnahmekammer bildende Öffnung umfasst, dort vollständig aufgenommen wird. Bei einer nachfolgenden Verschweißung der Metallfassung mit dem chirurgischen Instrument lässt sich eine dichte Verbindung zwischen der Metallfassung und dem chirurgischen Instrument herstellen, so dass die Aufnahmekammer des Gehäuses in Richtung der Öffnung offen bleiben kann. Die Metallfassung und deren Verbindung mit dem chirurgischen Instrument bildet einen ausreichenden Schutz gegen Umgebungseinflüsse, insbesondere auch beim Sterilisieren der Instrumente.

Bevorzugt wird das RFID-Element mit seiner Antenne der Metallfassung abgewandt und/oder von dieser räumlich beabstandet in der Aufnahmekammer angeordnet.

Die Verbindung zwischen dem Gehäuse und der Metallfassung kann in verschiedenen Variationen realisiert werden. Bei einer Variante wird das Gehäuse an die Metallfassung angespritzt. Hierbei kommen insbesondere Kunststoffmaterialien für die Herstellung des Gehäuses in Frage.

Andererseits kann auch die Metallfassung an das Gehäuse angespritzt werden, wobei hier das Gehäuse dann vorzugsweise aus Keramikmaterial gebildet wird.

Weiter alternativ kann das Gehäuse, hergestellt aus Kunststoff- oder Keramikmaterialien, an der Metallfassung kraft- und/oder formschlüssig, insbesondere auch im Press-Sitz gehalten werden.

Schließlich ist auch ein Verkleben des Gehäuses mit der Metallfassung, ggf. unter gleichzeitigem Verschließen der einen Zugang zur Aufnahmekammer bildenden Öffnung, möglich.

Bevorzugt weist das Gehäuse des RFID-Tags einen im Wesentlichen ebenen Oberflächenbereich auf, der eine vorgegebene Orientierung zu der Antenne des RFID-Elements aufweist, welche bevorzugt ungefähr koplanar mit der Antennenebene des RFID-Elements gewählt ist. So kann bei bereits geschlossenem Gehäuse eine korrektere Platzierung und Orientierung des RFID-Tags am Instrument erfolgen.

Die Erfindung betrifft des Weiteren ein Instrument bestückt mit einem RFID-Tag der vorliegenden Erfindung.

Insbesondere ist bevorzugt, wenn die Metallfassung des RFID-Tags an das Instrument angeschweißt wird, wobei hier insbesondere Laserschweißen bevorzugt wird.

Bei weiter bevorzugten erfindungsgemäßen Instrumenten ist das Instrument mit zwei oder mehr RFID-Tags bestückt, wobei die zwei oder mehr RFID-Tags so an dem Instrument angeordnet sind, dass die Antennen ihrer jeweiligen RFID-Elemente in unterschiedlichen Ebenen ausgerichtet sind, wobei die Ebenen vorzugsweise einen Winkel von ca. 70 ° bis ca. 110 ° miteinander bilden, vorzugsweise ca. 90 °.

Die Verwendung von zwei oder mehr RFID-Tags an einem Instrument erlaubt eine einfachere und sicherere Identifizierung des Instruments im Pulk mit anderen Instrumenten.

Bei einer besonders bevorzugten Ausführungsform der erfindungsgemäßen RFID-Tags sind zwei RFID-Elemente mit im Wesentlichen orthogonaler Ausrichtung der Antennen zueinander in einem gemeinsamen Gehäuse angeordnet. Hier kann dann eine lageunabhängige Identifizierung des Instruments mit nur einem RFID-Tag erfolgen.

Mit den erfindungsgemäß ausgebildeten RFID-Tags ist es insbesondere möglich, die Tags am Instrument selbst ohne eine Änderung des Instruments zu befestigen, insbesondere muss an dem Instrument keine Komponente angebohrt oder sonst wie verändert werden, um den RFID-Tag aufzunehmen.

Im Stand der Technik führt dies häufig zu Festigkeitsproblemen, darüber hinaus kann eine solche Änderung am Instrument selbst dazu führen, dass das Instrument seine Zulassung verliert, insbesondere dann, wenn die Bohrung nicht in den ursprünglichen Fertigungsunterlagen des Instruments definiert ist, d.h. wenn das Instrument nachträglich bestückt wird.

Damit ist auch aufgezeigt, dass sich die erfindungsgemäßen RFID-Tags v.a. auch dann mit Vorteil einsetzen lassen, wenn herkömmliche Instrumente, die nicht von vornherein für die Bestückung mit RFID-Tags entwickelt wurden, über RFID-Tags identifizierbar und nachverfolgbar gemacht werden sollen.

Darüber hinaus sind die RFID-Tags der vorliegenden Erfindung einfach mit großer Dichtigkeit herstellbar und sicher am Instrument anzubringen, wobei eine gute Reinigbarkeit, eine gute Funktion und eine hohe Flexibilität bei der Platzwahl für das Bestücken der Instrumente gegeben ist.

Diese und weitere Vorteile der vorliegenden Erfindung werden im Folgenden anhand der Zeichnung noch näher erläutert. Es zeigen im Einzelnen:
- Figur 1A: eine Explosionsdarstellung einer ersten Ausführungsform eines erfindungsgemäßen RFID-Tags;
- Figur 1B: eine Schnittzeichnung durch den zusammengesetzten RFID-Tag der ersten Ausführungsform der Figur 1A;
- Figur 2A: Explosionsdarstellung einer zweiten Ausführungsform des erfindungsgemäßen RFID-Tags;
- Figur 2B: eine Schnittzeichnung durch den zusammengebauten RFID-Tag der Figur 2A;
- Figur 2C: den RFID-Tag der Figur 2A an einem chirurgischen Instrument gehalten;
- Figur 3A: Explosionsdarstellung einer dritten Ausführungsform eines erfindungsgemäßen RFID-Tags;
- Figur 3B: Schnittdarstellung durch den zusammengebauten RFID-Tag der Figur 3A;
- Figur 4: ein chirurgisches Instrument mit einem erfindungsgemäßen RFID-Tag der Figur 1A;
- Figur 5: ein weiteres chirurgisches Instrument mit einem erfindungsgemäßen RFID-Tag der Figur 1A;
- Figur 6: ein weiteres chirurgisches Instrument mit einem erfindungsgemäßen RFID-Tag der Figur 1A;
- Figur 7: ein weiteres chirurgisches Instrument mit zwei erfindungsgemäßen RFID-Tags der Figuren 1A und 2C;
- Figur 8: ein weiteres chirurgisches Instrument, mit zwei RFID-Tags der Figur 1A bestückt; und
- Figur 9: ein weiteres chirurgisches Instrument, mit einem erfindungsgemäßen RFID-Tag der Figur 3A bestückt.

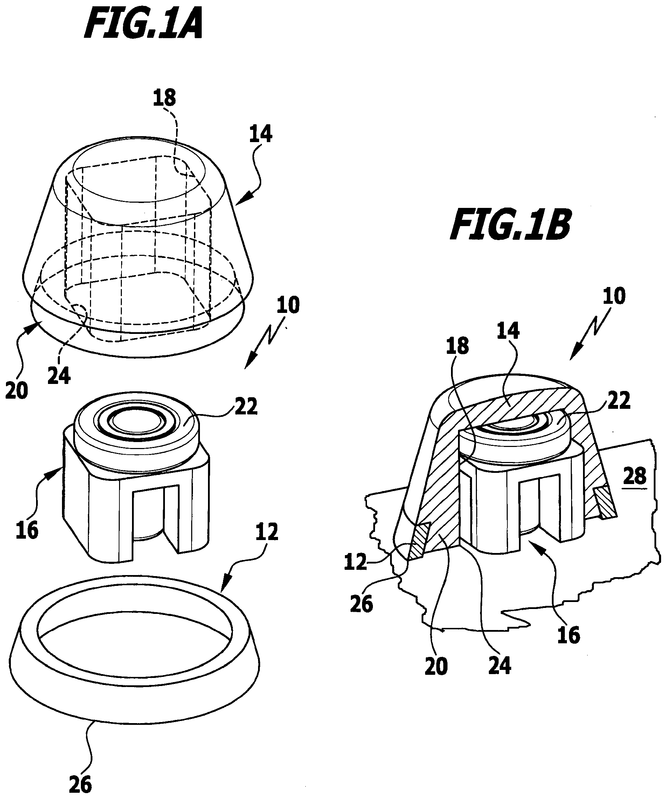

Figur 1 zeigt ein insgesamt mit dem Bezugszeichen 10 bezeichnetes RFID-Tag der vorliegenden Erfindung mit einer ringförmigen Metallfassung 12, einem Gehäuse 14 sowie einem in dem Gehäuse anzuordnenden RFID-Element 16.

Das Gehäuse 14 weist eine Aufnahmekammer 18 auf, in der das RFID-Element 16 aufgenommen wird.

Das Gehäuse 14 weist ferner eine Kegelstumpf-förmige Außenkontur auf mit einem am unteren Kegelstumpf-Ende zurückspringenden Rand 20, welcher in die komplementär gestaltete Öffnung der ringförmigen Metallfassung 12 im Press-Sitz eingesetzt werden kann. Letzteres ist möglich, wenn das Gehäuse 14 aus Kunststoff gefertigt ist. Alternativ kann der Metallring 12 auch an das Gehäuse 14, welches dann beispielsweise aus Keramikmaterial besteht, angespritzt werden.

Das erste Ende des Gehäuses 14 mit dem zurückspringenden Rand 20 weist darüber hinaus auch eine Öffnung 24 auf, die einen Zugang zur Aufnahmekammer 18 des Gehäuses 14 schafft.

Die Aufnahmekammer 18 und die Öffnung 24 des Gehäuses 14 sind der Geometrie des RFID-Elements 16 angepasst, so dass dieses geführt und mit vorgegebener räumlicher Orientierung in der Aufnahmekammer 18 platziert werden kann.

Das RFID-Element 16 wird vorzugsweise so in die Aufnahmekammer 18 eingefügt, dass dessen in Figur 1A oben gezeichnete Antenne 22 im montierten Zustand des RFID-Tags 10 von der ringförmigen Metallfassung 12 räumlich beabstandet ist und insbesondere von der Metallfassung abgewandt angeordnet ist. Der rückspringende Rand 20 des Gehäuses 14 bildet dessen erstes Ende, mit dem dieses in der ringförmigen Metallfassung 12 gehalten ist.

Das RFID-Element 16 wird in der Aufnahmekammer 18 entweder form- und/ oder kraftschlüssig oder aber stoffschlüssig mittels eines Klebers, insbesondere eines auf Silikonmaterial basierenden Klebers, befestigt. Alternativ ist es auch möglich, das Gehäuse 14 aus Kunststoff an das RFID-Element 16 in der gezeigten gegenseitigen Orientierung anzuspritzen.

Das erste Ende des Gehäuses 14 mit seiner Öffnung 24 kann im montierten Zustand des RFID-Tags 10 offen bleiben, da nachfolgend der RFID-Tag mit dem unteren Rand 26 der ringförmigen Metallfassung 12 an einem chirurgischen Instrument gehalten wird, vorzugsweise über eine ringsum laufende Laserschweißnaht, so dass das RFID-Element 16 ausreichend gegen Umgebungseinflüsse geschützt ist.

Figur 1B zeigt eine Schnittzeichnung des RFID-Tags 10 in zusammengebautem Zustand, wobei zur Verdeutlichung der Montage des RFID-Tags 10 auf einem Instrument eine Instrumentenoberfläche 28 schematisch noch mit abgebildet ist.

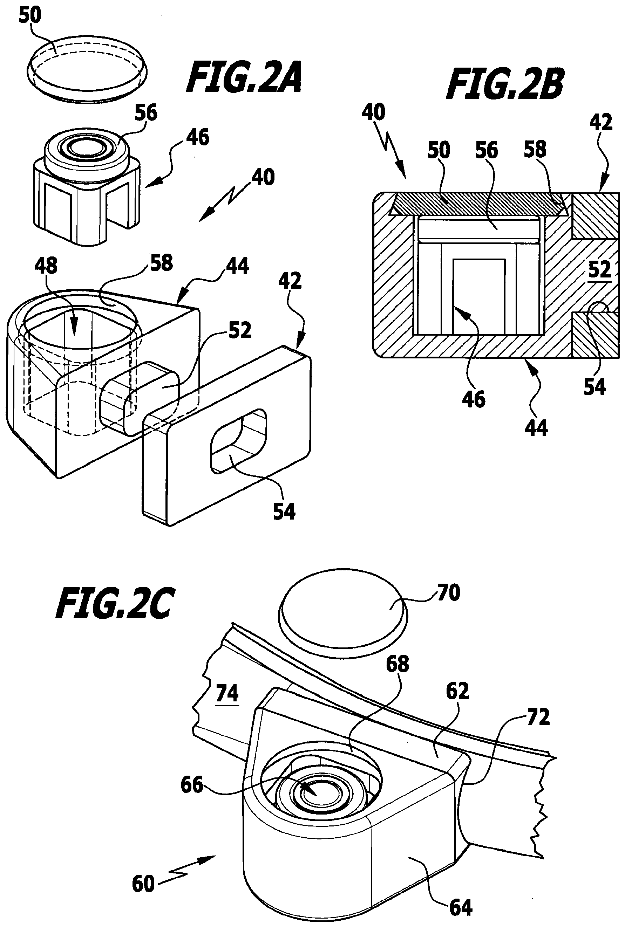

Figur 2A zeigt in einer Explosionsdarstellung eine zweite Ausführungsform eines erfindungsgemäßen RFID-Tags 40 mit einer hier rechteckig ausgebildeten Metallfassung 42, einem im Querschnitt parabelförmig ausgebildeten Gehäuse 44 sowie einem RFID-Element 46.

Das Gehäuse 44 weist eine einseitig offene Aufnahmekammer 48 auf, die der Form des RFID-Elements 46 wiederum angepasst ist. Die einseitig offene Aufnahmekammer 48 lässt sich bei eingesetztem RFID-Element 46 mittels eines Verschlusses oder Deckelelements 50 dichtend verschließen, beispielsweise durch Verkleben oder Verschweißen, insbesondere mit einem aus einem Kunststoffmaterial gefertigten Kleber.

Die Symmetrieachse der Aufnahmekammer 48 ist senkrecht zu der Parabelebene des Gehäuses ausgerichtet.

Das im Querschnitt parabelförmig ausgebildete Gehäuse 44 weist an seinem ersten Ende einen zapfenförmigen Vorsprung 52 auf, der in eine Öffnung 54 der Metallfassung 42 einrücken und form- und/oder kraftschlüssig oder auch stoffschlüssig, beispielsweise mittels eines Silikonmaterials verklebt, fixiert werden kann.

Wiederum ist das RFID-Element 46 mit einer Antenne 56 ausgerüstet, welche im montierten Zustand des RFID-Elements in dem Gehäuse 44 räumlich beabstandet von der Metallfassung 42 angeordnet ist.

Figur 2B zeigt das erfindungsgemäße RFID-Tag 40 im zusammengebauten Zustand, wobei in der Schnittzeichnung der Figur 2B ersichtlich ist, dass das Deckel- oder Verschlusselement 50 aufgrund der komplementären Ausbildung von dessen Randbereich formschlüssig in eine Öffnung 58 des Gehäuses 44 eingesetzt werden kann. Für eine entsprechende Auswahl der Materialien lassen sich der Deckel 50 und das Gehäuse verschweißen, insbesondere mittels Ultraschall verschweißen oder aber miteinander verkleben.

In dem in den Figuren 2A und 2B gezeigten Ausführungsbeispiel ist das RFID-Element 46 vollständig in dem Gehäuse 44 verkapselt und so vor Umwelteinflüssen, insbesondere auch beim Sterilisieren eines damit bestückten Instruments, geschützt.

Figur 2B zeigt wiederum deutlich den räumlichen Abstand der Antenne 56 des RFID-Elements 46 von der Metallfassung 42, eine Voraussetzung für die erheblich verbesserte Sendeleistung des erfindungsgemäßen RFID-Tags 40.

In Figur 2C ist eine Variante des RFID-Tags 40 als RFID-Tag 60 dargestellt mit einer Metallfassung 62, einem Gehäuse 64 und einem in dem Gehäuse 64 aufgenommenen RFID-Element 66. Die einseitig in dem Gehäuse 64 vorhandene Öffnung 68 ist hier noch offen, und ein Deckelelement 70 ist bereitgestellt, um die Öffnung 68 zu schließen und damit das RFID-Element 66 zu verkapseln.

Figur 2C zeigt darüber hinaus eine Anpassung der dem Gehäuse 64 entgegen gesetzten Oberfläche 72 der Metallfassung 62, die konkav ausgebildet ist, um direkt an eine konvexe Oberfläche 74 eines chirurgischen Instruments anschließend formschlüssig angelegt und dann beispielsweise mittels Laserschweißen verbunden zu werden.

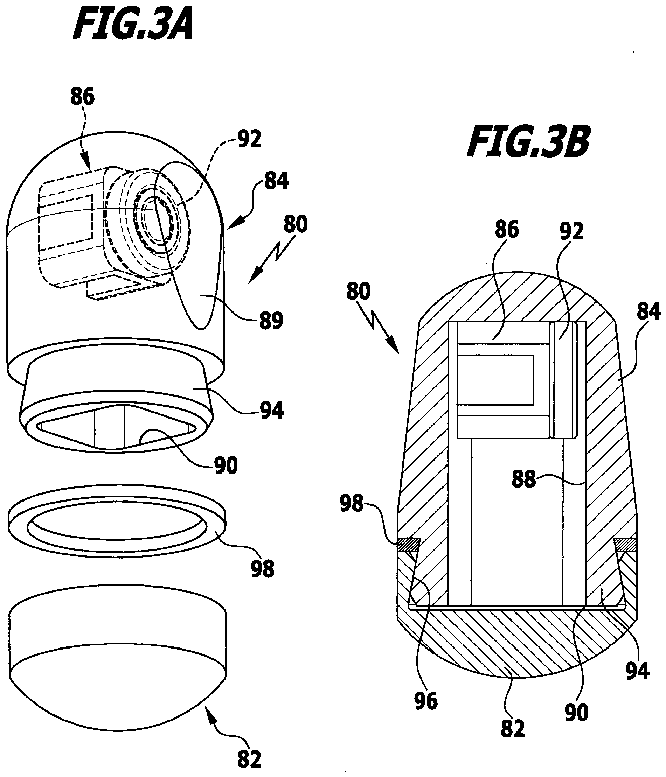

Die Figur 3A zeigt eine weitere Ausführungsform eines erfindungsgemäßen RFID-Tags 80 mit einer Metallfassung 82, einem Gehäuse 84 und einem in dem Gehäuse angeordneten RFID-Element 86.

Zur Aufnahme des RFID-Elements 86 weist das Gehäuse 84, das im Wesentlichen zylindrisch ausgebildet ist, eine Aufnahmekammer 88 auf, die sich parallel zur Zylinderachse des Gehäuses 84 erstreckt. Das Gehäuse 84 weist eine Öffnung 90 auf, die Zugang zu der Aufnahmekammer 88 schafft und über die das RFID-Element 86 in der Aufnahmekammer 88 eingesetzt werden kann. Das RFID-Element 86 kann in der Aufnahmekammer 88 beispielsweise durch Verklebung in seiner Position an dem ersten Ende bzw. dem der Öffnung 90 entgegengesetzten Ende fixiert werden, wie in Figur 3A und 3B dargestellt.

Dadurch wird die Antenne 92 des RFID-Elements 86 räumlich außerhalb der Metallfassung 82 und insgesamt deutlich von dieser beabstandet gehalten, so dass die Sendeleistung des RFID-Elements 86 nicht beeinträchtigt ist.

Ein im Wesentlichen planer Oberflächenbereich 89 des Gehäuses ist gegenüber der Position des RFID-Elements 86 mit seiner Antenne 92 koplanar orientiert, so dass auch im geschlossenen Zustand des RFID-Tags 80 die Orientierung der Antenne 92 noch erkennbar ist.

Am ersten Ende des Gehäuses 84, das die Öffnung 90 aufweist, ist ein rückspringendes konisches Strukturelement 94 angeordnet, welches in eine entsprechende als Sackloch ausgebildete Vertiefung 96 in der Metallfassung 82 einrücken kann.

Zur Abdichtung zwischen der Metallfassung 82 und dem Gehäuse 84 wird ein Dichtungselement 98 angeordnet, welches aufgrund der konischen Struktur des freien Endes 94 und der Öffnung 96 der Metallfassung 82, die hier eine Sacklochöffnung ist, eingespannt wird, so dass eine dichtende Verbindung zwischen dem Gehäuse 84 und der Metallfassung 82 gebildet wird. Auch hier wiederum ist das RFID-Element 86 vor Umgebungseinflüssen ausreichend geschützt.

Wie im Fall der zuvor beschriebenen erfindungsgemäßen RFID-Tags kann auch das RFID-Tag 80 mit seiner Metallfassung 82 an einem Instrument angeschweißt und so ein entsprechendes Instrument bestückt werden.

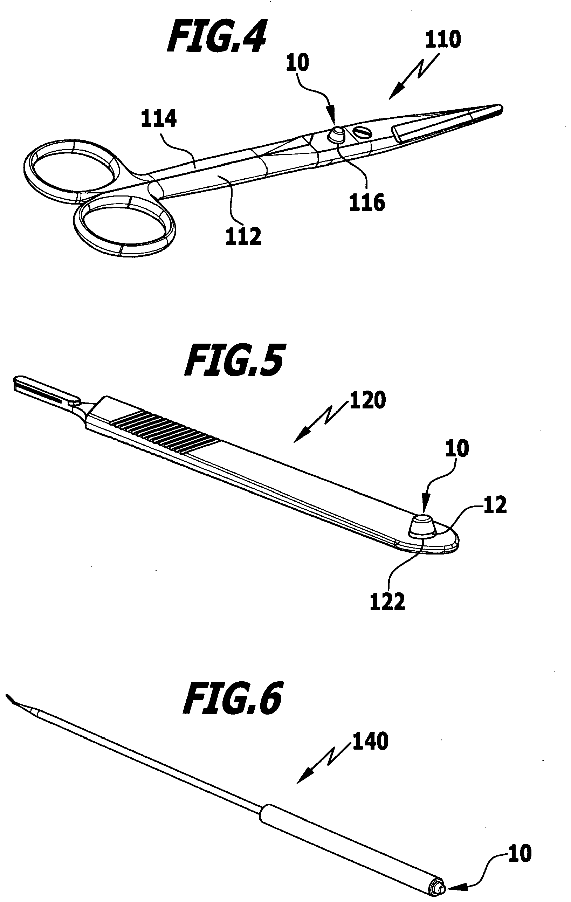

Die Figur 4 zeigt beispielhaft an einem Instrument in Form einer Schere 110 die Bestückung eines chirurgischen Instruments mit dem RFID-Tag 10 der Figur 1A. Hier ist das RFID-Tag 10 im Bereich der Schwenkverbindung zweier Branchen 112, 114 des Instruments 110 mittels einer Laserschweißnaht 116 fixiert. Gleichzeitig wird dadurch die Aufnahmekammer des RFID-Tags 10 dichtend abgeschlossen und so das RFID-Element innerhalb des RFID-Tags 10 vor Umgebungseinflüssen geschützt.

Figur 5 zeigt ebenfalls die Verwendung eines RFID-Tags 10 fixiert an einem Skalpellhalter 120. Erneut wurde das RFID-Tag 10 mit seiner Metallfassung 12 über eine umlaufende Laserschweißnaht 122 mit der Oberfläche des chirurgischen Instruments 120 dichtend verbunden.

Figur 6 zeigt die Befestigung eines RFID-Tags 10 an einem chirurgischen Instrument 140, das die Form eines Häkchens annimmt. Hier zeigt sich, dass die RFID-Tags der vorliegenden Erfindung sehr klein gebaut und auch an sehr kleinen Oberflächen sicher, d.h. abdichtend, befestigt werden können.

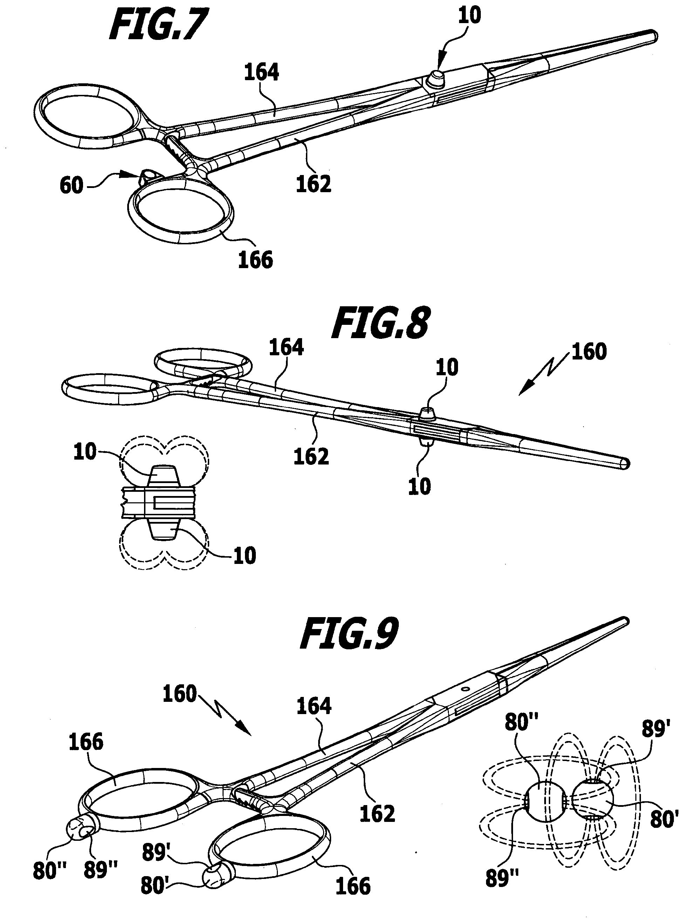

Figur 7 zeigt eine Klemme 160, bei der zum Einen ein RFID-Tag 10 im Bereich der gelenkigen Verbindung zweier Branchen 162, 164 auf einer ebenen Fläche aufgeschweißt ist. Darüber hinaus ist an einem Ring 166 am proximalen Ende der Griffbranche 162 ein weiterer RFID-Tag 60, wie er im Zusammenhang mit der Figur 2C beschrieben wurde, angeordnet, der unterstützend die Erkennung des Instruments 116 erleichtert, wenn dieses Instrument in einem Pulk mit anderen Instrumenten erfasst werden soll.

Figur 8 zeigt die Klemme 160 der Figur7, dieses Mal bestückt mit zwei RFID-Tags 10 an der Ober- und Unterseite der beiden Griffbranchen 162, 164. Damit kann der Effekt, dass das Instrument 160 selbst das Antennensignal abschattet, erheblich verringert werden. Die Abstrahlcharakteristik der beiden RFID-Tags 10 ist schematisch in Figur 8 dargestellt. Die RFID-Tags können so im Wesentlichen unabhängig von der Lage des Instruments ausgelesen werden.

Figur 9 zeigt schließlich die Klemme 160 mit zwei RFID-Tags 80', 80", wie er im Rahmen der Figuren 3A und 3B beschrieben wurde, ausgerüstet. Die RFID-Tags 80', 80" sind dabei an den Ringen 166, 168 der Griffbranchen 162 bzw. 164 angeordnet, wobei die Orientierung der RFID-Tags 80', 80" mit ihren Antennen orthogonal zueinander gewählt ist, wie dies einfach an der Orientierung der Flächen 89', 89" zueinander ersichtlich ist. Die Abstrahlcharakteristik der beiden RFID-Tags 80', 80" ist in Figur 9 schematisch zusätzlich gezeigt. Hier ist das Auslesen der RFID-Tags in jeder Position oder Lage des Instruments möglich.

## Patentansprüche

1. RFID-Tag (10; 40; 60; 80) zur Bestückung von chirurgischen Instrumenten, umfassend ein aus einem elektrisch nicht leitenden oder wenig leitfähigen Material hergestelltes Gehäuse (14; 44; 64; 84) mit einer Aufnahmekammer (18; 48; 68; 88), eine Metallfassung (12; 42; 62; 82) für das Gehäuse sowie ein RFID-Element mit einer Antenne (22; 56; 92), welches in der Aufnahmekammer (18; 48; 68; 88) des Gehäuses (14; 44; 64; 84) angeordnet ist, **dadurch gekennzeichnet, dass** das Gehäuse (14; 44; 64; 84) ein erstes Ende aufweist, welches in der Metallfassung (12; 42; 62; 82) gehalten ist, dass die Aufnahmekammer (18; 48; 68; 88) in dem Gehäuse von dessen erstem Ende räumlich beabstandet ausgebildet ist und dass das RFID-Element in der Aufnahmekammer (18; 48; 68; 88) so positioniert ist, dass dessen Antenne räumlich im Wesentlichen außerhalb der Metallfassung (12; 42; 62; 82) angeordnet ist und dass die Metallfassung (12; 42; 62; 82) an das chirurgische Instrument anschweißbar ausgebildet ist.

2. RFID-Tag (10; 40; 60; 80) nach Anspruch 1, **dadurch gekennzeichnet, dass** das Gehäuse aus einem Kunststoff- oder einem keramischen Material hergestellt ist.

3. RFID-Tag (10; 40; 60; 80) nach Anspruch 1 oder 2, **dadurch gekennzeichnet, dass** das Gehäuse ein einseitig offenes Gehäuse ist, wobei die Öffnung einen Zugang zu der Aufnahmekammer (18; 48; 68; 88) bildet.

4. RFID-Tag (10; 80) nach Anspruch 3, **dadurch gekennzeichnet, dass** das erste Ende die den Zugang zur Aufnahmekammer (18; 88) bildende Öffnung umfasst.

5. RFID-Tag (10) nach Anspruch 4, **dadurch gekennzeichnet, dass** das RFID-Element mit seiner Antenne (22) der Metallfassung (12) abgewandt in der Aufnahmekammer (18) angeordnet ist.

6. RFID-Tag (40; 60) nach einem der Ansprüche 1 bis 5, **dadurch gekennzeichnet, dass** die Aufnahmekammer mit einem Verschlusselement (50; 70) verschließbar ist, wobei das Verschlusselement vorzugsweise mit dem Gehäuse verklebt oder verschweißt, insbesondere mittels Ultraschall verschweißt, ist.

7. RFID-Tag nach einem der Ansprüche 1 bis 6, **dadurch gekennzeichnet, dass** das Gehäuse an die Metallfassung angespritzt ist.

8. RFID-Tag nach einem der Ansprüche 1 bis 6, **dadurch gekennzeichnet, dass** die Metallfassung an das Gehäuse angespritzt ist.

9. RFID-Tag nach einem der Ansprüche 1 bis 6, **dadurch gekennzeichnet, dass** das Gehäuse an der Metallfassung kraft- und/oder formschlüssig, weiter bevorzugt im Presssitz, gehalten ist.

10. RFID-Tag nach einem der Ansprüche 1 bis 9, **dadurch gekennzeichnet, dass** das RFID-Element in der Aufnahmekammer mittels eines Klebemittels fixiert ist, wobei das Klebemittel bevorzugt ein auf einem Silikonmaterial basierendes Klebemittel ist.

11. RFID-Tag (10; 40; 60; 80) nach einem der voranstehenden Ansprüche, **dadurch gekennzeichnet, dass** das Gehäuse (14; 44; 64; 84) einen im Wesentlichen planen Oberflächenbereich aufweist, dessen Ebene eine vorgegebene Orientierung zu der Antenne (22; 56; 92) des RFID-Elements aufweist.

12. RFID-Tag nach einem der voranstehenden Ansprüche, **dadurch gekennzeichnet, dass** in der Aufnahmekammer des Gehäuses zwei RFID-Elemente angeordnet sind, wobei diese gegeneinander so orientiert sind, dass die Ebenen ihrer Antennen einen Winkel von ca. 70 ° bis ca. 110 °, insbesondere ca. 90 ° miteinander bilden.

13. Chirurgisches Instrument (110; 120; 140; 160) bestückt mit einem RFID-Tag nach einem der Ansprüche 1 bis 12.

14. Chirurgisches Instrument nach Anspruch 13, **dadurch gekennzeichnet, dass** die Metallfassung an das Instrument insbesondere mittels Laserschweißen angeschweißt ist.

15. Chirurgisches Instrument (160) nach Anspruch 13 oder 14, **dadurch gekennzeichnet, dass** das Instrument mit zwei oder mehr RFID-Tags bestückt ist, wobei bevorzugt die zwei oder mehr RFID-Tags so an dem Instrument angeordnet sind, dass die Antennen ihrer jeweiligen RFID-Elemente in unterschiedlichen Ebenen ausgerichtet sind, wobei die Ebenen vorzugsweise einen Winkel von ca. 70 ° bis ca. 110 °, insbesondere ca. 90 °, miteinander bilden.

## Claims

1. An RFID tag (10; 40; 60; 80) for mounting on surgical instruments, comprising a housing (14; 44; 64; 84) that is made of an electrically non-conductive or slightly conductive material with an accommodating chamber (18; 48; 68; 88), a metal holder (12; 42; 62; 82) for the housing and an RFID element with an antenna (22; 56; 92), the RFID element being arranged in the accommodating chamber (18; 48; 68; 88) of the housing (14; 44; 64; 84), **characterized in that** the housing (14; 44; 64; 84) has a first end which is held in the metal holder (12; 42; 62; 82), **in that** the accommodating chamber (18; 48; 68; 88) in the housing (14; 44; 64; 84) is formed to be spatially spaced apart from the first end of the housing (14; 44; 64; 84), and **in that** the RFID element is positioned in the accommodating chamber (18; 48; 68; 88) in such a way that the antenna (22; 56; 92) of the RFID element is spatially arranged substantially outside of the metal holder (12; 42; 62; 82), and **in that** the metal holder (12; 42; 62; 82) is formed to be weldable onto the surgical instrument.

2. The RFID tag (10; 40; 60; 80) in accordance with Claim 1, **characterized in that** the housing is made of a plastics material or a ceramic material.

3. The RFID tag (10; 40; 60; 80) in accordance with Claim 1 or 2, **characterized in that** the housing is a housing that is open on one side, the opening forming an access to the accommodating chamber (18; 48; 68; 88).

4. The RFID tag (10; 80) in accordance with Claim 3, **characterized in that** the first end comprises the opening which forms the access to the accommodating chamber (18; 88).

5. The RFID tag (10) in accordance with Claim 4, **characterized in that** the RFID element is arranged in the accommodating chamber (18) with its antenna (22) facing away from the metal holder (12).

6. The RFID tag (40; 60) in accordance with any one of Claims 1 to 5, **characterized in that** the accommodating chamber is closable by a closure element (50; 70), wherein the closure element is glued or welded, in particular by means of ultrasound, to the housing.

7. The RFID tag in accordance with any one of Claims 1 to 6, **characterized in that** the housing is injection-molded onto the metal holder.

8. The RFID tag in accordance with any one of Claims 1 to 6, **characterized in that** the metal holder is injection-molded onto the housing.

9. The RFID tag in accordance with any one of Claims 1 to 6, **characterized in that** the housing is held on the metal holder in a form-fit and/or positive-fit manner, in particular by a press fit.

10. The RFID tag in accordance with any one of Claims 1 to 9, **characterized in that** the RFID element is fixed in the accommodating chamber by means of an adhesive, wherein the adhesive is in particular an adhesive that is based on a silicone material.

11. The RFID tag (10; 40; 60; 80) in accordance with any one of the preceding Claims, **characterized in that** the housing (14; 44; 64; 84) has a substantially planar surface region, the plane of which has a predefined orientation relative to the antenna (22; 56; 92) of the RFID element.

12. The RFID tag in accordance with any one of the preceding Claims, **characterized in that** two RFID elements are arranged in the accommodating chamber of the housing, these being oriented relative to one another in such a manner that the planes of their antennas form an angle of approximately 70° to approximately 110°, in particular 90°, with one another.

13. A surgical instrument (110; 120; 140; 160) equipped with an RFID tag according to any one of Claims 1 to 12.

14. The surgical instrument in accordance with Claim 13, **characterized in that** the metal holder is welded to the instrument, in particular by means of laser welding.

15. The surgical instrument (160) in accordance with Claim 13 or 14, **characterized in that** two or more RFID tags are mounted on the instrument, wherein it being preferred that the two or more RFID tags are arranged on the instrument in such a manner that the antennas of their respective RFID elements are aligned in different planes, wherein the planes in particular form an angle of approximately 70° to approximately 110°, in particular 90°, with one another.

## Revendications

1. Tag RFID (10; 40; 60; 80) destiné à équiper des instruments chirurgicaux, comprenant un boitier (14; 44; 64; 84) fabriqué en un matériau non conducteur ou peu conducteur sur le plan électrique et présentant une chambre d'accueil (18; 48; 68; 88), une monture métallique (12; 42; 62; 82) pour le boitier, ainsi qu'un élément RFID avec une antenne (22; 56; 92), qui est agencé dans la chambre d'accueil (18; 48; 68; 88) du boitier (14; 44; 64; 84), **caractérisé en ce que** le boitier (14; 44; 64; 84) présente une première extrémité qui est maintenue dans la monture métallique (12; 42; 62; 82), **en ce que** la chambre d'accueil (18; 48; 68; 88) est formée dans le boitier de manière à être éloignée spatialement de la première extrémité de celui-ci, et **en ce que** l'élément RFID est positionné dans la chambre d'accueil (18; 48; 68; 88) de manière à ce que son antenne soit agencée spatialement sensiblement à l'extérieur de la monture métallique (12; 42; 62; 82) et **en ce que** la monture métallique (12; 42; 62; 82) est agencée de manière soudable sur l'instrument chirurgical.

2. Tag RFID (10; 40; 60; 80) selon la revendication 1, **caractérisé en ce que** le boitier est fabriqué en une matière plastique ou en un matériau céramique.

3. Tag RFID (10; 40; 60; 80) selon la revendication 1 ou la revendication 2, **caractérisé en ce que** le boitier est un boitier ouvert d'un seul côté, l'ouverture formant un accès à la chambre d'accueil (18; 48; 68; 88).

4. Tag RFID (10; 80) selon la revendication 3, **caractérisé en ce que** la première extrémité comprend l'ouverture formant l'accès à la chambre d'accueil (18; 88).

5. Tag RFID (10) selon la revendication 4, **caractérisé en ce que** l'élément RFID est agencé dans la chambre d'accueil (18), avec son antenne (22) éloignée de la monture métallique (12).

6. Tag RFID (40; 60) selon l'une des revendications 1 à 5, **caractérisé en ce que** la chambre d'accueil peut être fermée à l'aide d'un élément de fermeture (50; 70), l'élément de fermeture étant de préférence collé ou soudé au boitier, notamment soudé par ultrasons.

7. Tag RFID selon l'une des revendications 1 à 6, **caractérisé en ce que** le boitier est moulé par injection sur la monture métallique.

8. Tag RFID selon l'une des revendications 1 à 6, **caractérisé en ce que** la monture métallique est moulée par injection sur le boitier.

9. Tag RFID selon l'une des revendications 1 à 6, **caractérisé en ce que** le boitier est maintenu sur la monture métallique par une liaison par adhérence et/ou complémentarité de formes, et de manière particulièrement préférée selon un montage à ajustement serré.

10. Tag RFID selon l'une des revendications 1 à 9, **caractérisé en ce que** l'élément RFID est fixé dans la chambre d'accueil au moyen d'une colle, la colle étant de préférence une colle à base de silicone.

11. Tag RFID (10; 40; 60; 80) selon l'une des revendications précédentes, **caractérisé en ce que** le boitier (14; 44; 64; 84) comporte une zone de surface sensiblement plane, dont le plan présente une orientation prédéterminée par rapport à l'antenne (22; 56; 92).

12. Tag RFID selon l'une des revendications précédentes, **caractérisé en ce que** dans la chambre d'accueil du boitier sont agencés deux éléments RFID, ceux-ci étant orientés l'un par rapport à l'autre de manière telle que les plans de leurs antennes forment entre eux un angle d'environ 70° à environ 110°, notamment d'environ 90°.

13. Instrument chirurgical (110; 120; 140; 160) équipé d'un tag RFID selon l'une des revendications 1 à 12.

14. Instrument chirurgical selon la revendication 13, **caractérisé en ce que** la monture métallique est soudée sur l'instrument, notamment par soudage laser.

15. Instrument chirurgical (160) selon la revendication 13 ou la revendication 14, **caractérisé en ce que** l'instrument est équipé de deux tags RFID ou davantage, lesdits deux tags RFID ou davantage étant de préférence agencés sur l'instrument de manière telle que les antennes de leurs éléments RFID respectifs soient orientées dans des plans différents, les plans formant de préférence entre eux un angle d'environ 70° à environ 110°, notamment d'environ 90°.
